# EUROPÄISCHE PATENTSCHRIFT

(11) **EP 3 682 968 B1**
(45) Veröffentlichungstag und Bekanntmachung des Hinweises auf die Patenterteilung: **29.05.2024**
(21) Anmeldenummer: 20151235.7
(22) Anmeldetag: 10.01.2020
(51) Int. Cl.: B01J 27/199, B01J 37/16, B01J 37/08, C07D 307/60

(54) **VERFAHREN ZUR HERSTELLUNG EINES VPO-KATALYSATORS**
METHOD FOR PREPARING A VPO CATALYST
PROCÉDÉ DE FABRICATION D'UN CATALYSEUR VPO

(30) Priorität: 16.01.2019 DE 102019100983
(43) Veröffentlichungstag der Anmeldung: 22.07.2020
(73) Patentinhaber: Clariant International Ltd, 4132 Muttenz (CH)
(72) Erfinder: BOECKLEIN, Sebastian, 83052 Heufeld (DE); MESTL, Gerhard, 80935 Muenchen (DE); BINDSEIL, Gabriele, 83052 Bruckmuehl (DE); HAUSMANN, Rene, 83043 Bad Aibling (DE); LIMBRUNNER, Sarah, 83052 Bruckmuehl (DE); WALDSCHUETZ, Anna, 83104 Tuntenhausen (DE)

(56) Entgegenhaltungen:
- US-A- 5 929 256
- US-A1- 2004 229 750

## Beschreibung

Die Erfindung betrifft ein Verfahren zur Herstellung eines VPO-Katalysators, enthaltend Molybdän und eine Vanadylpyrophosphat-Phase, umfassend die Schritte:
a) Bereitstellen einer Reaktionsmischung, die V₂O₅ als eine V(V)-Verbindung, Phosphorsäure mit einer Konzentration zwischen 98 und 100% als eine P(V)-Verbindung, eine Mo-Verbindung, einen aromatischen Alkohol als Reduktionsmittel und einen aliphatischen Alkohol als Lösungsmittel umfasst und die zwischen 50 und 80 Gew.-% Lösungsmittel, zwischen 5 und 15 Gew.-% Reduktionsmittel, zwischen 5 und 15 Gew.-% V(V)-Verbindung, zwischen 5 und 15 Gew.-% P (V)-Verbindung und zwischen 0,05 bis 0,3 Gew.-% Mo-Verbindung, jeweils bezogen auf das Gesamtgewicht der Mischung, enthält,
b) Reduzieren der V(V)-Verbindung mit dem Reduktionsmittel zumindest in Teilen zu Vanadylhydrogenphosphat, um eine Zwischenproduktsuspension zu erhalten,
c) Filtrieren der Zwischenproduktsuspension aus Schritt b), um ein Zwischenprodukt zu erhalten,
d) Trocknen des Zwischenprodukts bei einer Temperatur von maximal 350 °C, um ein getrocknetes Zwischenprodukt zu erhalten und
e) Aktivieren des getrockneten Zwischenprodukts bei einer Temperatur oberhalb 200 °C,
dadurch gekennzeichnet, dass in Schritt a) maximal 0,2 Gew.-% Wasser, bezogen auf das Gewicht der Reaktionsmischung, anwesend ist und während der Reduktion in Schritt b) kein Wasser entzogen wird.

Die Erfindung betrifft des Weiteren einen VPO-Katalysator, der durch das erfindungsgemäße Verfahren herstellbar ist und der Molybdän und eine Vanadylpyrophosphat-Phase enthält sowie die Verwendung des erfindungsgemäßen Katalysators zur Oxidation von Butan zu Maleinsäureanhydrid.

Maleinsäureanhydrid ist ein chemisches Zwischenprodukt von großer wirtschaftlicher Bedeutung. Es wird beispielsweise bei der Herstellung von Alkyd- und Polyesterharzen allein oder auch in Kombination mit anderen Säuren eingesetzt. Darüber hinaus stellt es auch ein vielseitig einsetzbares Zwischenprodukt für die chemische Synthese dar, zum Beispiel für die Synthese von γ-Butyrolacton, Tetrahydrofuran und 1,4-Butandiol, welche wiederum ihrerseits als Lösungsmittel eingesetzt werden oder zu Polymeren, wie beispielsweise Polytetrahydrofuran oder Polyvinylpyrrolidon, weiterverarbeitet werden können.

Die Herstellung von Maleinsäureanhydrid erfolgt in der Regel durch partielle Oxidation von Butan in der Gasphase mit molekularem Sauerstoff oder mit einem molekularen Sauerstoff enthaltenden Gas in Gegenwart eines Vanadium-Phosphor-Oxid-Katalysators (VPO-Katalysators) der Vanadylpyrophosphat (VPP) enthält. Vanadylpyrophosphat weist in Reinform Vanadium mit einer Wertigkeit von + 4 auf und ist besonders geeignet für die Herstellung von Maleinsäureanhydrid aus unverzweigten gesättigten oder ungesättigten Kohlenwasserstoffen mit mindestens vier Kohlenstoffatomen. Zum Einsatz kommen sowohl Festbettreaktoren als auch Wirbelschichtreaktoren.

VPO-Katalysatoren weisen nur eine geringe intrinsische Aktivität bei der Reaktion von n-Butan zu Maleinsäureanhydrid auf. Daher ist für eine ausreichende Umwandlung eine große Menge an Katalysator erforderlich. Bei den VPO-Katalysatoren kommt noch hinzu, dass sie zu den teuersten Nicht-Edelmetallkatalysatoren überhaupt zählen, im Wesentlichen auf Grund der hohen Kosten ihrer Ausgangsmaterialien. Infolgedessen stellt sich die Aufgabe, die Katalyse-Performance (Aktivität und Selektivität) bzw. auch Lebensdauer solcher Katalysatoren zu verbessern. Aus dem Stand der Technik ist bekannt, dass man die Performance der VPO-Katalysatoren durch die Beigabe von Fremdelementen zur VPO-Phase verbessern kann, wie zum Beispiel durch die Beigabe von Molybdän (Mo-Promotor oder Mo-Dotierung).

US 5,929,256 offenbart die Synthese eines aktiven mit Molybdän modifizierten Vanadium-Phosphor-Katalysators zur Herstellung von Maleinsäureanhydrid. Hierbei wird eine in wesentlichen Teilen 5-wertiges Vanadium enthaltende Verbindung mit einer 5-wertigen Phosphor enthaltenden Verbindung in einem alkoholischen Medium, das zur Reduktion des Vanadiums auf eine Oxidationsstufe unter 5 geeignet ist, umgesetzt. Hierbei wird Molybdän in das Reaktionsprodukt eingebaut, wobei eine feste, mit Molybdän modifizierte Vorläuferzusammensetzung gebildet wird. Der Alkohol wird entfernt, um eine getrocknete feste mit Molybdän modifizierte Vorläuferzusammensetzung zu erhalten. Formkörper, die die getrocknete feste mit Molybdän modifizierte Vorläuferverbindung enthalten, werden geformt. Die getrockneten und geformten mit Molybdän modifizierten Vorläuferzusammensetzungen werden aktiviert, um sie in den aktiven Katalysator umzuwandeln.

DE 10 2014 004786 A1 betrifft einen Katalysator, der ein Vanadium-Phosphor-Oxid und ein Alkalimetall enthält, worin der Gewichtsanteil an Alkalimetall im Vanadium-Phosphor-Oxid im Bereich von 10 bis 400 ppm liegt, bezogen auf das Gesamtgewicht des Vanadium-Phosphor-Oxids, ein Verfahren zu dessen Herstellung sowie die Verwendung des Katalysators zur Gasphasenoxidation von Kohlenwasserstoffen, insbesondere zur Herstellung von Maleinsäureanhydrid.

Um VPO-Katalysatoren welche eine VPP-Phase enthalten herzustellen, wird üblicherweise eine Reduktion von Vanadiumpentoxid (V₂O₅) bei gleichzeitiger Anwesenheit von Phosphorsäure (PPA) in einem organischen alkoholischen Lösungsmittel mit Benzylalkohol als Reduktionsmittel durchgeführt, wobei neben Benzaldehyd Vanadylhydrogenphosphat (VHP) entsteht. Die dabei ablaufende Redoxreaktion (die "Reduktion"), bei der Vanadium mit der Oxidationsstufe V (V(V)) zur VHP-Phase reagiert, in der Vanadylspezies (VO²⁺) mit Vanadium in der Oxidationsstufe IV (V(IV)) vorliegen, lautet:

(1) V₂O₅ + 2H₃PO₄ + Ph-CH₂-OH → 2VOHPO₄ * ½H₂O + Ph-CHO + 2H₂O

In einem darauffolgenden Aktivierungsschritt wird durch die Einwirkung von Wärme die VHP-Phase unter Abspaltung von Wasser zur Vanadylpyrophosphat-Phase umgewandelt.

(2) 2VOHPO₄ * ½H₂O → (VO)₂P₂O₇ + 1½H₂O

Wie aus der Reaktionsgleichung (1) ersichtlich ist, werden in dem Reduktionsschritt zwei Äquivalente Wasser frei. Selbst wenn man bei dieser organischen Route zur VHP-Katalysatorsynthese wasserfreies Lösungsmittel, üblicherweise Isobutanol (IBA), sowie wasserfreie Reaktanden verwendet, führt das während der ablaufenden Reaktion entstehende Wasser zu einer zunehmenden Verdünnung der Reaktionsmischung mit Wasser. Bislang ist man davon ausgegangen, dass die Anwesenheit des entstehenden Wassers nachteilig ist und die Performance des so erhaltenen Katalysators erniedrigt. Dementsprechend werden bislang während der Reduktion Mittel eingesetzt, um das entstandene Wasser zu entfernen, entweder physikalisch, z.B. mit Hilfe eines Wasserabscheiders, oder chemisch, durch die Verwendung von Verbindungen, die das entstehende Wasser binden, also z.B. Anhydriden, wie Phosphorsäure mit mehr als 100 % Konzentration.

Überraschenderweise wurde gefunden, dass speziell bei VPO-Katalysatoren, die Molybdän und eine Vanadylpyrophosphat-Phase aufweisen, die Anwesenheit des durch die Reduktion entstehenden Wassers für die Katalysatorperformance förderlich ist, insbesondere dann, wenn zu Beginn der Reduktion kein Wasser anwesend ist. Insbesondere positiv beeinflusst wird die Effektivität des Molybdänpromotors zur Reduzierung der Nebenprodukte, darunter vor allem Essigsäure (AcA) und Acrylsäure (AcrA).

DE 2611290 A1 betrifft einen Vanadium-Phosphor-Sauerstoff-Katalysator-Komplex, welcher dadurch gekennzeichnet ist, dass er als aktive Hauptkomponenten Vanadium, Phosphor und Me im Atomverhältnis von 1:0,90 bis 1,3:0,005 bis 0,4 umfasst, wobei Me U, W oder eine Mischung von Elementen ausgewählt aus der Gruppe bestehend aus Zn , Cr, U, W, Cd, Ni, B und Si darstellt. Offenbart wird auch ein Verfahren zur Herstellung von Maleinsäureanhydrid, dadurch gekennzeichnet, dass man eine Beschickung von normal-C4-Kohlenwasserstoffen in der Dampfphase bei erhöhten Temperaturen mit Sauerstoff und dem Vanadium-Phosphor-Sauerstoff-Katalysator-Komplex kontaktiert.

Aufgabe der vorliegenden Erfindung war es daher, einen verbesserten VPO-Katalysator zur Gasphasenoxidation von Kohlenwasserstoffen, insbesondere zur Herstellung von Maleinsäureanhydrid bereitzustellen, der eine verbesserte Katalysatorperformance, d.h. eine verbesserte Aktivität und Selektivität durch verringerte Nebenproduktbildung, und eine verbesserte Stabilität aufweist sowie ein Verfahren zu dessen Herstellung.

Diese Aufgabe wird gelöst durch ein Verfahren zur Herstellung eines VPO-Katalysators, enthaltend Molybdän und eine Vanadylpyrophosphat-Phase, umfassend die Schritte:
a) Bereitstellen einer Reaktionsmischung, die V₂O₅ als eine V(V)-Verbindung, Phosphorsäure mit einer Konzentration zwischen 98 und 100% als eine P(V)-Verbindung, eine Mo-Verbindung, einen aromatischen Alkohol als Reduktionsmittel und einen aliphatischen Alkohol als Lösungsmittel umfasst und die zwischen 50 und 80 Gew.-% Lösungsmittel, zwischen 5 und 15 Gew.-% Reduktionsmittel, zwischen 5 und 15 Gew.-% V(V)-Verbindung, zwischen 5 und 15 Gew.-% P (V)-Verbindung und zwischen 0,05 bis 0,3 Gew.-% Mo-Verbindung, jeweils bezogen auf das Gesamtgewicht der Mischung, enthält,
b) Reduzieren der V(V)-Verbindung mit dem Reduktionsmittel zumindest in Teilen zu Vanadylhydrogenphosphat, um eine Zwischenproduktsuspension zu erhalten,
c) Filtrieren der Zwischenproduktsuspension aus Schritt b), um ein Zwischenprodukt zu erhalten,
d) Trocknen des Zwischenprodukts bei einer Temperatur von maximal 350 °C, um ein getrocknetes Zwischenprodukt zu erhalten und
e) Aktivieren des getrockneten Zwischenprodukts bei einer Temperatur oberhalb 200 °C,
dadurch gekennzeichnet, dass in Schritt a) maximal 0,2 Gew.-% Wasser, bezogen auf das Gewicht der Reaktionsmischung, anwesend ist und während der Reduktion in Schritt b) kein Wasser entzogen wird.

Die V(V)-Verbindung, die als Ausgangsmaterial in der Reaktionsmischung nach Schritt a) verwendet wird, ist V₂O₅.

Die P(V)-Verbindung, die als Ausgangsmaterial in der Reaktionsmischung nach Schritt a) verwendet wird, ist Phosphorsäure mit einer Konzentration von 98 bis 100%, bevorzugt 99 bis 100% (die Angabe bezieht sich auf den üblicherweise angegebenen prozentualen Gewichtsanteil von Phosphorsäure in Bezug zu dem Gewicht der Wasser-Phosphorsäuremischung). Alternativ kann Phosphorsäure mit mehr als 100 % eingesetzt werden, die mit dem in der Reaktionsmischung nach Schritt a) zu Beginn eventuell vorhandenen Wasser zu Phosphorsäure mit einer Konzentration von 98 bis 100 %, bevorzugt 99 bis 100 %, vorzugsweise 100 % reagiert, so dass in der Reaktionsmischung nach Schritt a) keine Phosphorsäure mit mehr als 100 % Konzentration vorliegt und gleichzeitig nicht mehr als 0,2 Gew.-% Wasser, bezogen auf das Gewicht der Reaktionsmischung, in der Reaktionsmischung verbleibt.

Die Mo-Verbindung, die als Ausgangsmaterial in der Reaktionsmischung nach Schritt a) verwendet wird, ist eine beliebige Verbindung, die Molybdän enthält, zum Beispiel Molybdäntrioxid, Ammoniumheptamolybdat ((NH₄)₆Mo₇O₂₄)*4H₂O), Ammoniumparamolybdat ((NH₄)₆Mo₇O₂*4H₂O), meta Molybdat, Molybdänsäure (H₂MoO₄) sowie deren Salze wie (NH₄)₂MoO₄, Na₂MoO₄, oder K₂MoO₄.

Das Reduktionsmittel, das in der Reaktionsmischung nach Schritt a) anwesend ist, ist ein aromatischer Alkohol, insbesondere Benzylalkohol.

Das Lösungsmittel, das in der Reaktionsmischung nach Schritt a) anwesend ist, ist ein hochsiedender aliphatischer Alkohol, insbesondere Isobutanol, alternativ Ethanol oder iso-Propanol.

Die Reaktionsmischung nach Schritt a) enthält zwischen 50 und 80 Gew.-% Lösungsmittel, zwischen 5 und 15 Gew.-% Reduktionsmittel, zwischen 5 und 15 Gew.-% V(V)-Verbindung, zwischen 5 und 15 Gew.-% der P (V)-Verbindung und zwischen 0,05 bis 0,3 Gew.-% der Mo-Verbindung, jeweils bezogen auf das Gesamtgewicht der Mischung. Vorzugsweise enthält die Reaktionsmischung nach Schritt a) zwischen 60 und 70 Gew.-% Isobutanol, zwischen 5 und 15 Gew.-% Benzylalkohol, zwischen 5 und 15 Gew.-% V₂O₅, zwischen 10 und 20 Gew.-% Phosphorsäure und zwischen 0,1 und 0,2 Gew.-% eines Molybdat-Salzes, wie Ammoniumdimolybdat, jeweils bezogen auf das Gesamtgewicht der Reaktionsmischung.

Erfindungsgemäß ist in der Reaktionsmischung nach Schritt a), also vor dem Reduktionsschritt b), maximal 0,2 Gew.-% Wasser, bezogen auf das Gewicht der Reaktionsmischung, anwesend. Gegebenenfalls muss mit geeigneten Maßnahmen sichergestellt werden, dass nicht mehr 0,2 Gew.-% Wasser, bezogen auf das Gewicht der Reaktionsmischung anwesend ist, dies kann zum Beispiel mit Hilfe eines Wasserabscheiders, oder chemisch durch die Verwendung von Verbindungen, die das Wasser binden, also z.B. Anhydriden wie Phosphorsäure mit mehr als 100 % Konzentration erfolgen. Bevorzugt ist, dass die Reaktionsmischung nach Schritt a) maximal 0,15 Gew.-% Wasser, besonders bevorzugt weniger als 0,10 Gew.-% Wasser, noch stärker bevorzugt weniger als 0,05 Gew.-% Wasser, am stärksten bevorzugt weniger als 0,01 Gew.-% Wasser, jeweils bezogen auf das Gesamtgewicht der Reaktionsmischung, aufweist. Die Reaktionsmischung nach Schritt a) kann auch zwischen 0,05 Gew.-% Wasser und 0,2 Gew.-% Wasser, bevorzugterweise 0,1 bis 0,15 Gew.-% Wasser enthalten. Die Reaktionsmischung nach Schritt a) kann auch innerhalb der Nachweisgrenze wasserfrei sein.

Im Verfahrensschritt b) erfolgt die zumindest teilweise Reduktion der V(V)-Verbindung zu einem Zwischenprodukt, das Molybdän und eine Vanadylhydrogenphosphat-Phase enthält und das mit dem Lösungsmittel und den übrigen Komponenten aus Schritt a), die Zwischenproduktsuspension bildet. Diese Reduktion erfolgt vorzugsweise unter Reflux bei Normaldruck, hierbei ist die Temperatur in Abhängigkeit des Siedepunkts des verwendeten Lösungsmittels erhöht, bevorzugterweise wird ausschließlich ein einziger Refluxschritt in dem erfindungsgemäßen Verfahren durchgeführt. Das Zwischenprodukt enthält vorzugsweise als Hauptphase Vanadylhydrogenphosphat oder kann sogar im Wesentlichen aus Vanadylhydrogenphosphat-Phase bestehen. Das in dem Zwischenprodukt ebenfalls anwesende Molybdän kann als Dotierung der Vanadylhydrogenphosphat-Phase vorliegen, wobei unter Molybdändotierung zu verstehen ist, dass das Molybdän entweder in die Vanadylhydrogenphosphat-Phase eingebaut wird oder auf dessen Oberfläche vorliegt. Neben der Vanadylhydrogenphosphat-Phase können bei der Reduktion jedoch noch weitere Vanadium-Phosphor-Mischoxide entstehen, in denen Vanadium eine Oxidationsstufe von IV oder sogar III aufweist. Die Reduktion muss nicht vollständig verlaufen, so dass auch Anteile der V(V)-Verbindung und der P(V)-Verbindung in dem Zwischenprodukt und damit in der Zwischenproduktsuspension verbleiben. Typischerweise weist das Zwischenprodukt der Reduktion aber eine mittlere Oxidationsstufe des Vanadiums von 3,8 bis 4,2 auf.

Das während der Reduktion entstehende Wasser soll erfindungsgemäß während der Reduktion nicht aus der Reaktionsmischung entfernt werden, d.h. anders als im Stand der Technik werden zum Zeitpunkt der Reduktion keine Mittel eingesetzt, die Wasser entfernen. Die Entfernung des Wassers während der Reduktion erfolgt im Stand der Technik entweder physikalisch, z.B. mit Hilfe eines Wasserabscheiders oder chemisch durch die Verwendung von Verbindungen, die das Wasser binden, also z.B. Anhydriden wie Phosphorsäure mit mehr als 100 % Konzentration. Erfindungsgemäß weist die Reaktionsmischung nach Schritt a) keine Anhydride auf, die mit Wasser reagieren, und dieses binden, insbesondere weist die Reaktionsmischung nach Schritt a) keine Phosphorsäure mit mehr als 100 % Konzentration auf.

Der Verfahrensschritt c), d.h. die Filtration der Zwischenproduktsuspension, erfolgt durch die dem Fachmann bekannten Mittel, typischerweise durch eine Filterpresse, einen Dekanter oder durch eine Nutsche.

Das Trocknen des durch die Filtration erhaltenen Zwischenprodukts (der feste Rückstand der Filtration) in dem Schritt d), erfolgt bei einer Temperatur oberhalb der Raumtemperatur, typischerweise bei einer Temperatur bis zu 300 °C bei reduziertem Druck, bzw. Vakuum oder unter Inertgas, wie Stickstoff oder einem Edelgas. Bevorzugterweise verläuft das Trocknen in zwei Schritten d₁) Trocknen im Vakuum zwischen 90 °C und 140 °C und d₂) Trocknen in Stickstoff zwischen 230 und 300 °C.

Optional können sich an den Trocknungsschritt d) ein oder mehrere der Verfahrensschritt(e):
d₃) Beimischen von 1 bis 10 Gew.-% Graphit zu dem Zwischenprodukt, um ein Zwischenproduktgemisch zu erhalten,
d₄) Kompaktieren und/oder Granulieren des Zwischenproduktes oder des Zwischenproduktgemisches, und
d₅) Tablettieren des Zwischenproduktes oder des Zwischenproduktgemisches anschließen.

Im Verfahrensschritt e), erfolgt die Aktivierung des erhaltenen Zwischenprodukts, bei einer Temperatur oberhalb von 200 °C. Die Aktivierung erfolgt typischerweise in einer Gasmischung bestehend aus Luft, Stickstoff und Wasserdampf, bei einer Temperatur im Bereich von 300 °C bis 500 °C, vorzugsweise im Bereich von 350 °C bis 450 °C. Mit der Aktivierung wird der fertige Katalysator als Endprodukt erhalten. Sofern das graphithaltige Zwischenproduktgemisch aktiviert wird, wird ein graphithaltiger Katalysator als Endprodukt erhalten. Das tablettierte Endprodukt hat typischerweise eine Seitendruckfestigkeit von 15 bis 45 N.

Das Endprodukt ohne Graphit weist Vanadylpyrophosphat mit einem Überschuss an Phosphat auf, so dass sich eine Stöchiometrie von (VO)₂P₂O₇MoₘP₂ₚO_{y} ergibt, mit m zwischen 0,003 bis 0,03, vorzugsweise 0,006 bis 0,02, besonders bevorzugt zwischen 0,012 und 0,019, p zwischen 0 bis 0,2 und y einen Wert annimmt, um die Ladungsneutralität zu gewähren (bei einer mittleren Oxidationsstufe des Vanadiums von 4,0 und p = 0 ist y = 0).
Figur 1: Auftragung des Butanumsatzes als Funktion der Kontaktzeit zur Ermittlung der Katalysatoraktivität.
Figur 2: Auftragung des Butanumsatzes gegen die Selektivität zu Maleinsäureanhydrid.

### Beispiele

Es wurde eine Reihe an Synthesen unter Variation der Wasseranteile vor bzw. während des Refluxschrittes durchgeführt.

Beispiel 1 ist ein erfindungsgemäßes Beispiel, hier nimmt die Wasserkonzentration durch das in der Synthese entstehende Wasser mit Verlauf der Reaktion zu. Beispiel 2 soll Beispiel 1 unter Verwendung billigerer Ausgangsstoffe (105 % PPA und 98.5% IBA) nachstellen, wobei davon ausgegangen wird, dass die 105% PPA mit dem im IBA enthaltenen Wasser zu 100% PPA und reinem IBA reagiert. Beispiele 3 und 4 sollen den Einfluss von Wasser zu Beginn der Synthese zeigen (in etwa die gleiche Wasserkonzentration wie nach abgelaufener Reaktion). In Beispiel 5 wird unter Verwendung eines Wasserabscheiders die Wasserkonzentration während des gesamten Refluxvorgangs konstant gehalten.

### Beispiel 1 (Erfindung)

### Laborsynthese des Endprodukts

Auf einen Laborboy wird ein Heizpilz gestellt und in diesem befindet sich ein 2L-Vierhalskolben. In der mittleren Öffnung des Vierhalskolbens befindet sich ein Halbmondrührer mit passendem Rührverschluss, der mittels einer Rührerkupplung an das Rührwerk angeschlossen ist. In der rechten Öffnung befindet sich ein Thermometer, in der Linken ein Steigrohr zum Rückflusskühler. Die Öffnung vorne in der Mitte wird zum Befüllen mit den Chemikalien verwendet, danach wird die Stickstoffspülung dort angeschlossen. Die gesamte Apparatur kann auch mit Stickstoff geflutet werden. Hierfür wird der Stickstoff als erstes durch eine Gaswaschflasche und dann in die Apparatur geleitet und oben aus dem Kühler wiederum durch eine Gaswaschflasche ausgeleitet.

### Herstellen der Reaktionsmischung und Reduktion

Als erstes werden 1069,5 g Isobutanol und 156,0 g Benzylalkohol zugegeben. Unter Rühren erfolgt die Zugabe von 150 g V₂O₅. Nach der V₂O₅ Zugabe erfolgt die Zugabe von 2,52 g Ammoniumdimolybdat. Anschließend werden 232,50 g Phosphorsäure (100 %, bzw. wasserfrei) zur Suspension zugegeben und unter N₂ im Rückfluss für 10 h geheizt.

### Filtrieren:

Nach Abkühlen der Zwischenproduktsuspension wird diese aus dem Vierhalskolben in eine Filternutsche übertragen und die Flüssigkeit abgesaugt. Der feuchte Filterkuchen wird in einer Presse über Nacht bei 14 bis 18 bar trocken gepresst.

### Trocknung:

Der ausgepresste Filterkuchen wird in den Verdampferkolben eines Rotationsverdampfers gefüllt. Unter Wasserstrahlvakuum wird der Filterkuchen bei 110 °C über Nacht getrocknet. Das so getrocknete Pulver wird in einem geeigneten Kalziniertopf in einen Ofen gestellt und in einer N₂-Atmosphäre bei Temperaturen von 200 bis 300 °C für 9 Stunden kalziniert. Es wird das getrocknete Zwischenprodukts (VMo_{0,0088}OHPO₄ × 0,5 H₂O)

### Tablettierung:

Vor der Kompaktierung/Tablettierung werden dem kalzinierten pulverförmigen Zwischenprodukt 5 Gew.-% Graphit zugegeben und mit Hilfe eines Rhönradmischers homogen durchmischt. Dieses Pulver wird mit einem Walzenkompaktor mit einem Anpressdruck von 190 bar, einer Spaltbreite 0,60 mm und einer Walzengeschwindigkeit von 7 U/min, zu Platten kompaktiert und durch ein 1 mm Sieb granuliert.

Das Granulat wird mit einer Rundläufertablettenpresse zu der gewünschten Tablettenform, mit entsprechender Höhe, z.B. 5,6 × 5,6 × 2,3 mm, und Seitendruckfestigkeit, gepresst.

### Aktivierung zum Pyrophosphat:

Die Aktivierung, bei der Vanadiumpyrophosphat entsteht, wird in einer in einem programmierbaren Ofen eingebauten Retorte unter kontrollierten Bedingungen durchgeführt. Die kalzinierten Tabletten werden gleichmäßig in die Retorte eingefüllt und diese wird dicht verschlossen. Danach wird der Katalysator in einer feuchten Luft-Stickstoffmischung (50% absolute Luftfeuchtigkeit) zuerst bei über 300 °C für 5 h anschließend bei über 400 °C für 9 h aktiviert.

### Beispiel 2 (Erfindung)

Die Synthese wurde wie in Beispiel 1 durchgeführt, jedoch wurde 105%ige Phosphorsäure (PPA) anstelle von 100% PPA sowie Isobutanol mit 1 Gew.-% an destilliertem Wasser anstelle von wasserfreien Isobutanol verwendet (nach Einwaagen ergeben sich formal wieder reines Isobutanol und 100% PPA).

### Beispiel 3 (Vergleich)

Die Synthese wurde wie in Beispiel 1 durchgeführt, jedoch wurden dem hochreinen Isobutanol 2 Gew.-%, bezogen auf das Gewicht des Isobutanols, an destilliertem Wasser zugegeben.

### Beispiel 4 (Vergleich)

Die Synthese wurde wie in Beispiel 1 durchgeführt, jedoch wurden dem hochreinen Isobutanol 5 Gew.-%, bezogen auf das Gewicht des Isobutanols, an destilliertem Wasser zugegeben

### Beispiel 5 (Vergleich)

Die Synthese wurde wie in Beispiel 1 durchgeführt, jedoch wurde ein Wasserabscheider in die Refluxapparatur integriert, der während des Refluxschrittes der Reaktion Wasser entzog.

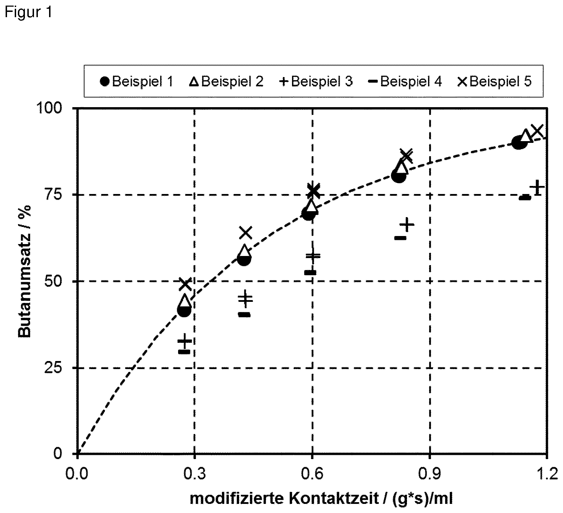

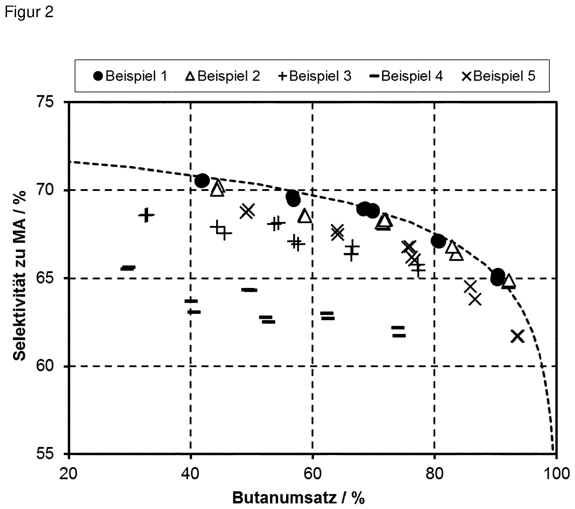

Zur Ermittlung der Katalysatorperformance wurden alle Katalysatoren nach erfolgter vollständiger Präparation (Reflux, Filtration, Vakuumtrocknung, Kalzinierung, Kompaktierung, Tablettierung, Aktivierung) in einem "Bench-Scale"-Reaktor bei 1,5 mol-% Butan in Luft in einem verdünnten Katalysatorbett (1:9 Mischung Katalysator: keramische Inertringe) hinsichtlich ihrer katalytischen Eigenschaften getestet. Zur Ermittlung der Katalysatoraktivität wurde die Reaktionsgeschwindigkeitskonstante kₐ bei einer Reaktionstemperatur von 410°C durch Auftragung des Butanumsatzes gegen die modifizierte Verweilzeit (Quotient aus Katalysatormasse und Eingangsvolumenstrom - die sonst übliche Kontaktzeit kann nicht verwendet werden, da es sich um Messungen an einem verdünnten Katalysatorbett handelt) unter Annahme einer Kinetik 1. Ordnung bestimmt. Abbildung 1 zeigt die experimentellen Daten, sowie eine Anpassung an Beispiel 1 unter Annahme einer Kinetik 1. Ordnung (gestrichelte Kurve). Die Selektivität zu Maleinsäureanhydrid (MA) wurde für den technisch relevanten Referenzumsatz von 85% aus den experimentellen Daten durch Auftragung der Selektivität gegen den Butanumsatz ermittelt (Figur 2). Ebenso wurde auch für die Nebenprodukte Essigsäure und Acrylsäure verfahren. Die Ergebnisse der Evaluation der Katalysatorperformance sind in Tabelle 1 beschrieben.

**Tabelle 1: Zusammenfassung der Testergebnisse hinsichtlich Aktivität (kₐ), MA-, AcA- und AcrA-Selektivität bei 85 % Butanumsatz.**

| **Beispiel** | **Refluxbedingungen** | ***k*ₐ [mL/(g*s)]** | **S_{MA} @ 85% X** | **S_{AcA} @ 85% X** | **S_{AcrA} @ 85% X** |
|---|---|---|---|---|---|
| 1 | < 0,2 % Wasser* | 2.0 | 66.5 | 0.18 | 0.17 |
| 2 | < 0,2 % Wasser* | 2.1 | 66.5 | 0.22 | 0.28 |
| 3 | + 2 % Wasser* | 1.3 | 64.3 | 0.20 | 0.14 |
| 4 | + 5 % Wasser* | 1.2 | 59.8 | 0.18 | 0.14 |
| 5 | Wasserabscheider | 2.4 | 64.7 | 0.20 | 0.17 |

| | | | | | |
|---|---|---|---|---|---|
| *Gew.-% Wasser bezogen auf das Gewicht der Reaktionsmischung vor der Reduktion. | | | | | |

## Patentansprüche

1. Verfahren zur Herstellung eines VPO-Katalysators, enthaltend Molybdän und eine Vanadylpyrophosphat-Phase, umfassend die Schritte:
a) Bereitstellen einer Reaktionsmischung, die V₂O₅ als eine V(V)-Verbindung, Phosphorsäure mit einer Konzentration zwischen 98 und 100% als eine P(V)-Verbindung, eine Mo-Verbindung, einen aromatischen Alkohol als Reduktionsmittel und einen aliphatischen Alkohol als Lösungsmittel umfasst und die zwischen 50 und 80 Gew.-% Lösungsmittel, zwischen 5 und 15 Gew.-% Reduktionsmittel, zwischen 5 und 15 Gew.-% V(V)-Verbindung, zwischen 5 und 15 Gew.-% P (V)-Verbindung und zwischen 0,05 bis 0,3 Gew.-% Mo-Verbindung, jeweils bezogen auf das Gesamtgewicht der Mischung, enthält,
b) Reduzieren der V(V)-Verbindung mit dem Reduktionsmittel zumindest in Teilen zu Vanadylhydrogenphosphat, um eine Zwischenproduktsuspension zu erhalten,
c) Filtrieren der Zwischenproduktsuspension aus Schritt b), um ein Zwischenprodukt zu erhalten,
d) Trocknen des Zwischenprodukts bei einer Temperatur von maximal 350 °C, um ein getrocknetes Zwischenprodukt zu erhalten und
e) Aktivieren des getrockneten Zwischenprodukts bei einer Temperatur oberhalb 200 °C,
**dadurch gekennzeichnet, dass** in Schritt a) maximal 0,2 Gew.-% Wasser, bezogen auf das Gewicht der Reaktionsmischung, anwesend ist und während der Reduktion in Schritt b) kein Wasser entzogen wird.

2. Verfahren nach Anspruch 1, **dadurch gekennzeichnet, dass** die Reaktionsmischung nach Schritt a) keine Verbindung aufweist, die mit Wasser reagiert und dieses bindet, insbesondere weist die Reaktionsmischung nach Schritt a) keine Phosphorsäure mit mehr als 100 % Konzentration auf.

3. Verfahren nach Anspruch 1 oder 2, **dadurch gekennzeichnet, dass** die P(V)-Verbindung 100%ige (wasserfreie) Phosphorsäure ist.

4. Verfahren nach einem der vorstehenden Ansprüche, **dadurch gekennzeichnet, dass** das Reduktionsmittel Benzylalkohol ist.

5. Verfahren nach einem der vorhergehenden Ansprüche, **dadurch gekennzeichnet, dass** das Lösungsmittel Isobutanol ist.

6. Verfahren nach einem der vorhergehenden Ansprüche, **dadurch gekennzeichnet, dass** die Reduktion unter Reflux bei Normaldruck durchgeführt wird.

7. Verfahren nach einem der vorhergehenden Ansprüche, **dadurch gekennzeichnet, dass** die Trocknung im Vakuum erfolgt.

8. Verfahren nach einem der vorhergehenden Ansprüche, umfassend den Schritt des Mischens des getrockneten Zwischenprodukts aus Schritt d) mit 2 bis 10 Gew.-% Graphit, bezogen auf das Gesamtgewicht des Gemisches und des Granulierens des Gemisches, um ein Granulat zu erhalten.

9. Verfahren nach einem der vorhergehenden Ansprüche, umfassend den Schritt des Tablettierens des getrockneten Zwischenprodukts oder des Granulats, um Tabletten zu erhalten.

10. Verfahren nach einem der vorhergehenden Ansprüche, umfassend den Schritt des Aktivierens des getrockneten Zwischenprodukts oder der Tabletten bei einer Temperatur oberhalb 200 °C in einem Gasgemisch enthaltend Stickstoff.

11. Verfahren nach einem der vorhergehenden Ansprüche, **dadurch gekennzeichnet, dass** in Schritt a) 0,001 bis 0,15 Gew.-%, bevorzugt 0,01 bis 0,1 Gew.-% Wasser, bezogen auf das Gesamtgewicht der Reaktionsmischung, anwesend ist.

12. VPO-Katalysator herstellbar durch ein Verfahren gemäß einem der Ansprüche 1 bis 11.

13. Verwendung des VPO-Katalysators nach Anspruch 12, zur Oxidation von Butan zu Maleinsäureanhydrid.

## Claims

1. A process for preparing a VPO catalyst containing molybdenum and a vanadyl pyrophosphate phase comprising the steps of:
a) Providing a reaction mixture comprising V₂O₅ as a V(V) compound, phosphoric acid having a concentration between 98 and 100% as a P(V) compound, a Mo compound, an aromatic alcohol as a reducing agent, and an aliphatic alcohol as a solvent, and comprising between 50 and 80 wt.% solvent, between 5 and 15 wt.% reducing agent, between 5 and 15 wt.% V(V) compound, between 5 and 15 wt.% P(V) compound, and between 0.05 to 0.3 wt.% Mo compound, each based on the total weight of the mixture,
b) Reducing the V(V) compound with the reducing agent at least in part to vanadyl hydrogen phosphate to obtain an intermediate product suspension,
c) Filtering the intermediate product suspension from step b) to obtain an intermediate product,
d) Drying the intermediate product at a maximum temperature of 350 °C to obtain a dried intermediate product and
e) Activation of the dried intermediate product at a temperature above 200 °C,
**characterized in that** a maximum of 0.2 wt. % of water, based on the weight of the reaction mixture, is present in step a) and no water is removed during the reduction in step b).

2. Process according to claim 1, **characterized in that** the reaction mixture after step a) does not have any compound which reacts with water and binds it, in particular the reaction mixture after step a) does not have any phosphoric acid with a concentration of more than 100 %.

3. Process according to claim 1 or 2, **characterized in that** the P(V) compound is 100% (anhydrous) phosphoric acid.

4. Process according to one of the preceding claims, **characterized in that** the reducing agent is benzyl alcohol.

5. Process according to one of the preceding claims, **characterized in that** the solvent is isobutanol.

6. Process according to one of the preceding claims, **characterized in that** the reduction is carried out under reflux at normal pressure.

7. Process according to one of the preceding claims, **characterized in that** the drying takes place in a vacuum.

8. A process according to any one of the preceding claims, comprising the step of mixing the dried intermediate product from step d) with 2 to 10% wt.% of graphite based on the total weight of the mixture and granulating the mixture to obtain a granulate.

9. A method according to any one of the preceding claims, comprising the step of tableting the dried intermediate product or granulate to obtain tablets.

10. A process according to any one of the preceding claims, comprising the step of activating the dried intermediate product or tablets at a temperature above 200°C in a gas mixture containing nitrogen.

11. Process according to one of the preceding claims, **characterized in that** in step a) 0.001 to 0.15 wt.%, preferably 0.01 to 0.1 wt.% of water, based on the total weight of the reaction mixture, is present.

12. VPO catalyst producible by a process according to any one of claims 1 to 11.

13. Use of the VPO catalyst according to claim 12, for the oxidation of butane to maleic anhydride.

## Revendications

1. Procédé de préparation d'un catalyseur VPO contenant du molybdène et une phase de pyrophosphate de vanadyle, comprenant les étapes consistant à:
a) Fournir un mélange réactionnel comprenant V₂O₅ en tant que composé V(V), acide phosphorique à une concentration entre 98 et 100% en tant que composé P(V), un composé Mo, un alcool aromatique en tant qu'agent réducteur et un alcool aliphatique en tant que solvant, et comprenant entre 50 et 80% en poids de solvant, entre 5 et 15 % en poids d'agent réducteur, entre 5 et 15 % en poids de composé V(V), entre 5 et 15 % en poids de composé P(V) et entre 0,05 et 0,3 % en poids de composé Mo, chacun sur la base du poids total du mélange,
b) la réduction du composé V(V) avec l'agent réducteur, au moins en partie, en hydrogénophosphate de vanadyle, pour obtenir une suspension intermédiaire,
c) filtrer la suspension intermédiaire de l'étape b) pour obtenir un produit intermédiaire,
d) le séchage du produit intermédiaire à une température maximale de 350 °C afin d'obtenir un produit intermédiaire séché, et
e) Activation du produit intermédiaire séché à une température supérieure à 200 °C,
**caractérisé en ce que** dans l'étape a), un maximum de 0,2 % en poids d'eau, par rapport au poids du mélange réactionnel, est présent et qu'aucune eau n'est extraite pendant la réduction dans l'étape b).

2. Procédé selon la revendication 1, **caractérisé en ce que** le mélange réactionnel après l'étape a) ne présente pas de composé qui réagit avec l'eau et la lie, en particulier le mélange réactionnel après l'étape a) ne présente pas d'acide phosphorique à une concentration supérieure à 100 %.

3. Procédé selon la revendication 1 ou 2, **caractérisé en ce que** le composé P(V) est de l'acide phosphorique à 100 % (anhydre).

4. Procédé selon l'une quelconque des revendications précédentes, **caractérisé en ce que** l'agent réducteur est l'alcool benzylique.

5. Procédé selon l'une des revendications précédentes, **caractérisé en ce que** le solvant est l'isobutanol.

6. Procédé selon l'une des revendications précédentes, **caractérisé en ce que** la réduction est effectuée sous reflux à pression normale.

7. Procédé selon l'une des revendications précédentes, **caractérisé en ce que** le séchage est effectué sous vide.

8. Procédé selon l'une des revendications précédentes, comprenant l'étape de mélange du produit intermédiaire séché de l'étape d) avec 2 à 10 % en poids de graphite par rapport au poids total du mélange et de la granulation du mélange pour obtenir un granulé.

9. Procédé selon l'une des revendications précédentes, comprenant l'étape de mise en comprimés du produit intermédiaire séché ou des granulés, pour obtenir des comprimés.

10. Procédé selon l'une des revendications précédentes, comprenant l'étape d'activation du produit intermédiaire séché ou des comprimés à une température supérieure à 200°C dans un mélange gazeux contenant de l'azote.

11. Procédé selon l'une des revendications précédentes, **caractérisé en ce que** dans l'étape a), de 0,001 à 0,15 % en poids, de préférence de 0,01 à 0,1 % en poids d'eau, par rapport au poids total du mélange réactionnel, est présent.

12. Catalyseur VPO pouvant être préparé par un procédé selon l'une des revendications 1 à 11.

13. Utilisation du catalyseur VPO selon la revendication 12, pour l'oxydation du butane en anhydride maléique.
